# EUROPEAN PATENT SPECIFICATION

(11) **EP 0 652 946 B1**
(45) Date of publication and mention of the grant of the patent: **26.01.2005**
(21) Application number: 94915572.5
(22) Date of filing: 27.04.1994
(51) Int. Cl.: C12N 9/20, C11D 3/386

(54) **NEW LIPASE VARIANTS FOR USE IN DETERGENT APPLICATIONS**
Neuartige Lipasevarianten zur Verwendung in Reinigungsmitteln
NOUVEAUX VARIANTS DE LIPASE UTILISES DANS DES DETERGENTS

(30) Priority: 27.04.1993 EP 93201212
(43) Date of publication of application: 17.05.1995
(73) Proprietor: GENENCOR INTERNATIONAL, INC., Palo Alto, California 94304 (US)
(72) Inventor: VAN DER LAAN, Jan, Metske, NL-4839 AP Breda (NL); LENTING, Hermanus, Bernardus, Maria, NL-2641 RT Pijnacker (NL); MULLENERS, Leonadrus, Johannes, Sofie, Marie, NL-5121 SV Rijen (NL); COX, Maria, Mathilde, Josephina, NL-2613 SN Delft (NL)
(74) Representative: Kiddle, Simon John
(86) International application number: PCT/EP1994/001435
(87) International publication number: WO 1994/025578

(56) References cited:
- EP-A- 0 407 225
- WO-A-87/00859
- WO-A-91/16423

## Description

### Technical Field

The present invention relates to modified lipases and to their use in, for example, detergents or cleaning compositions.

### Background of the Invention

Lipases are enzymes capable of hydrolyzing lipids, they are utilized in a wide range of applications such as fats and oil processing, detergents, diagnostic reagents etc.

Extracellular lipases (triacylglycerol acylhydrolases, E.C. 3.1.1.3) are produced by a wide variety of microorganisms. Suitable microbial lipases have for example been disclosed in U.S. Patent No. 3,950,277. These lipases were obtained from such diverse microorganisms as Pseudomonas, Aspergillus, Pneumococcus, Staphylococcus, Mycobacterium tuberculosis, Mycotorula lipolytica and Sclerotinia.

Examples of the use of lipase in detergent compositions are given in, e.g., EP 463100 (Pseudomonas alcaligenes), EP 0218272 (Pseudomonas pseudoalcaligenes), EP 0214761 (Pseudomonas cepacia)), EP 0258068 (Thermomyces), EP 206390 (Pseudomonas chromobacter, Pseudomonas fluorescens, Pseudomonas fragi, Pseudomonas nitroreducens, Pseudomonas gladioli and Chromobacter viscosum).

Pseudomonas lipases in particular appear to have favorable characteristics for desired applications. Pseudomonas species therefore have been extensively used for obtaining lipases. To increase lipase yield in fermentation several lipase genes have been cloned and expressed in both homologous and heterologous host strains. Examples of Pseudomonas species from which lipase gene cloning has been reported are Pseudomonas cepacia (EP 331376), Pseudomonas glumae (EP 464922), Pseudomonas pseudoalcaligenes (EP 334462), Pseudomonas fragi (EP 318775).

The classification of Pseudomonas species is based on DNA-rRNA and DNA-DNA hybridization studies as reported by Palleroni et al., Int. J. Syst. Bacteriol. 23:333 (1973). A more extensive overview can be found in Bergey's Manual of Systematic Bacteriology (Vol. 1, Section 4, pp. 160-161 (1984), Eds N.R. Krieg and J.G. Holt, Williams and Wilkins, Baltimore/London). This overview also reports that the classification is supported by morphological data and by 16S ribosomal RNA homology.

The described Pseudomonas strains, with favorable application characteristics, can be divided into two DNA homology groups. Pseudomonas pseudoalcaligenes, Pseudomonas alcaligenes, Pseudomonas aeruginosa (see e.g. EP 0334462), Pseudomonas stutzeri and Pseudomonas mendocina are closely related and belong to Group I. For example, Pseudomonas aeruginosa lipase has 81% homology with Pseudomonas pseudoalcaligenes lipase. Pseudomonas glumae and Pseudomonas cepacia belong to Group II. In Table 1 a homology comparison of lipase genes, derived from different strains, is shown. From this homology comparison, it can be learned that the lipase derived from Pseudomonas fragi is situated between Group I and II.

There are differences between lipases originating from Group I and II. It was found that lipases belonging to Pseudomonas Group I have an increased hydrophobic character in comparison to those belonging to Group II and therefore are more lipophilic. This property is very beneficial for application in detergents. The hydrophobic character of Pseudomonas pseudoalcaligenes lipase results in an easy adherence to surfaces. Lipases from other groups or organisms e.g. Humicola lanuginosa, are often more hydrophilic. The high hydrofobicity of lipases from Group I can only be properly handled when detergents are present.

**Table 1**

| Homology to other Pseudomonas lipases | | | | | | |
|---|---|---|---|---|---|---|
| No. | Strain | 1 | 2 | 3 | 4 | 5 |
| 1 | P. pseudoalcaligenes | 100 | 71 | 40 | 38 | 41 |
| 2 | P. aeruginosa | 81 | 100 | 37 | 41 | 36 |
| 3 | P. cepacia | 52 | 41 | 100 | 33 | 78 |
| 4 | P. fragi | 56 | 51 | 46 | 100 | 34 |
| 5 | P. glumae | 59 | 41 | 82 | 52 | 100 |

The lower part of the Table shows a nucleic acid sequence comparison. The upper part shows an amino acid sequence comparison.

As already mentioned lipases can be used as ingredient in detergent compositions. Detergent compositions may contain, apart from lipase, many known ingredients depending on their formulation.
- Detergent powders generally contain builders (e.g. zeolite, phosphate), surfactants (e.g. anionic, nonionic) polymers (e.g. acrylic), bleach precursors (e.g. borate), bleach activators, structurant (e.g. silicate), pH adjusting compounds (e.g. alkali).
- Detergent liquids generally contain surfactants (e.g. anionic and nonionic), bleach precursors (e.g. borate), bleach activators, pH adjusting compounds (e.g. alkali).

Other ingredients such as enzymes (e.g. protease, amylase), organic acids, inorganic salts, fabric softeners can be incorporated into such compositions as well.

In order to be used as an ingredient in detergent compositions lipases have, apart from wash performance, to be resistant to the other ingredients present in such compositions.

Enzymes which are selected for application in detergent formulations can be developed or found in several ways, for example by classical screening methods or by using modern genetic and protein engineering techniques.

Screening for organisms or microorganisms that display the desired enzymatic activity, can be performed for example by isolating and purifying the enzyme from a microorganism or from a culture supernatant of such microorganisms, determining its biochemical properties and checking whether these biochemical properties meet the demands for a particular use. If the identified enzyme cannot be obtained from its natural producing organism, recombinant-DNA techniques may be used to isolate the gene encoding the enzyme, express the gene in another organism, isolate and purify the expressed enzyme and test whether it is suitable for the intended use.

Another way of obtaining new enzymes for an intended use is the modification of existing enzymes. This can be achieved inter alia by chemical modification methods (see WO 91/16423). In general these methods are too unspecific in that they modify all accessible residues with common side chains, or they are dependent on the presence of suitable amino acids to be modified, and are often unable to modify amino acids which are difficult to reach, unless the enzyme molecule is unfolded.

Alternatively, enzyme modification through mutagenesis of the encoding gene does not suffer from the aspecificities mentioned above, and therefore is thought to be superior. Mutagenesis can be achieved either by random mutagenesis or by site-directed mutagenesis.

Random mutagenesis by treating whole microorganisms with chemical mutagens or with mutagenizing radiation may of course result in modified enzymes. In this case strong selection protocols to search for these particular, rare mutants have to be available. Higher probability of isolating mutant enzymes by random mutagenesis can be achieved, after cloning the encoding gene, by mutagenizing it in vitro or in vivo and expressing the encoded enzyme by recloning of the mutated gene in a suitable host cell. Suitable hosts for the production of the modified enzymes are, for example, bacteria (E. coli, Bacillus, pseudomonas), yeasts or fungi (Aspergillus). Also in this case suitable biological selection protocols must be available in order to select the desired mutant enzymes. These biological selection protocols do not necessarily select directly the enzymes which are best suited for industrial application. EP 0407225, for example, describes a lipase with improved stability against attack by protease or oxidizing agents. It is true that these improvements may resolve specific problems, but they do not necessarily result in suitable or even better wash performance (see e.g. EP 0328229).

EP 0407225 further discloses mutations in the sequence of lipase from Pseudomonas glumae. This application does not teach which residue(s) should be replaced in case of genes encoding lipase originating from other microorganisms. In general the best choice of mutation for a specific enzyme is not automatically the best choice for another homologous enzyme and certainly not for other much less related enzymes.

WO 92/05249 describes lipase mutants with different hydrophobicity or electrostatic properties of a lipid contact zone which could lead to improved wash performance, stability, storage stability and specific activity. However, as the lipases described in this application do show little homology with the Pseudomonas lipases andy really exhibit quite different properties, application WO 92/05249 can not be used to decide which position should be mutated in order to obtain Pseudomonas lipases with desired properties.

### Summary of the Invention

The present invention now provides new modified Pseudomonas Group I lipases whereby the methionine at a position corresponding to position 21 in wild-type Pseudomonas pseudoalcaligenes lipase is substituted by leucine or alanine resulting in a desired property change. In a preferred embodiment of the invention these lipase variants exhibit improved wash performance under application conditions.

### Brief Description of the Figures

- Figure 1:: Site directed mutagenesis using PCR
- Figure 2:: Integration of plasmid pBR flank into chromosome
- Figure 3:: Recombination to delete lipase gene and plasmid from chromosome
- Figure 4:: Introduction of mutant lipase DNA into chromosome and plasmid
- Figure 5:: Recombination at 3' flanking region

### Detailed Description and Preferred Embodiments

As used in this specification, the term "improved wash performance" is used to indicate an improved wash result under application conditions, i.e. in the presence of a fully built detergent composition and at established conditions.

The methionine residue at position 16 in the lipase derived from Pseudomonas aeruginosa (Wohlfarth et al. (1992), J. of General Microbiology 138 (7): 1325-1335) is equivalent to the methionine on position 21 of the corresponding enzyme, derived from Pseudomonas pseudoalcaligenes, when the amino acid sequences are aligned and compared.

The wild-type lipase is a lipase producible by a Pseudomonas Group I microorganism, such a wild-type lipase has preferably 70% or more homology with Pseudomonas pseudoalcaligenes lipase. Homology is defined as the number of amino acids being identical when different amino acid sequences (or proteins) are aligned and compared.

We have for the first time succeeded to obtain mutant or modified enzymes which show suitable or even improved wash performance under industrial process conditions.

Site directed mutagenesis enabling specific substitution of one or more amino acids by any other desired amino acid can be used to construct and further select an enzyme with improved properties.

In more detail, specific mutations at a position corresponding to position 21 in Pseudomonas pseudoalcaligenes lipase, wherein the original methionine is replaced by leucine or alanine surprisingly show improved wash performance in the presence or absence of bleaching agents, indicating the importance of this position for wash performance. Thus, methionine substitution at position 21 not only results in enhanced oxidation stability, but also in improved wash performance.

The Pseudomonas pseudoalcaligenes lipase is closely related to other Pseudomonas lipases belonging to Group I of the Pseudomonas strains based on the high amino acid homology. This implies that the overall three-dimensional structure of these lipases are very similar. Therefore modifications on this structural position in the other lipases belonging to Group I of the Pseudomonas strains will also be functional with respect to the wash performance.

Amino acids suitable in the replacement of said methionine include Ala and Leu.

In a preferred embodiment of the invention the original methionine at position 21 corresponding to wild-type Pseudomonas pseudoalcaligenes lipase is replaced by leucine, which surprisingly shows improved performance, although no substantial changes in hydrophobicity or electrostatic properties appear in this mutant.

The invention also provides a process which comprises effecting a mutation in DNA encoding a Pseudomonas Group I lipase at a position corresponding to Met +21 in Pseudomonas pseudoalcaligenes lipase, and testing for a desired property change in the enzyme resulting from said mutation. Such property change may comprise improved wash performance, altered specific activity, altered pH activity, altered substrate activity or enhanced oxidation stability, or any combination thereof.

According to another aspect of the invention a selection process is provided which enables the selection of mutants with enhanced wash performance.

### Assay for the determination of the lipase performance under washing conditions (SLM-test).

For the purpose of this invention the so-called SLM test is used for the evaluation of alkaline lipase mutants in the washing process. The SLM test uses the same principles as the method developed by T. Hashimoto et al., Yukagaku 34 (1985), 606-612, but the time necessary for the analysis has been drastically reduced. The method includes using immobilized, non-emulsified fat or oil on a fabric as the test stain, extracting the swatch after the washing process and analysing the extracts for fats and fatty acids. Depending on the conditions used, fatty acids, formed as a result of lipase activity, together with residual triglycerides may stay on the textile during the washing process. Therefore, the quantities of the product left on the swatch appear to be a good measure of the performance of lipases during the washing process.

The following is a typical example of how the SLM test is preferably carried out. Polyester swatches are used as the fabric and triolein or purified olive oil (both products of Sigma, USA) as the substrates. The hydrolysis of triolein can be followed by chromatographic methods after extraction of textile.

The washing procedure preferably employed for the purpose of the SLM test is as follows: a volume of 80 µl containing 10 mg olive oil dissolved in n-hexane (12.5%) is spotted on a polyester swatch (3x3 cm). The swatch is air dried at room temperature. The washing solution consisting of 10 ml of STW (standard tap water: 2mM calcium chloride and 0.7 mM magnesium chloride in distilled water) or detergent dissolved in STW is placed in an Erlenmeyer flask (50 ml) with a ground stopper and kept in a shaking waterbath at 40°C. The washing process is started by adding lipase M1 (40 ILU, see below) and immediately thereafter the soiled swatch, to the Erlenmeyer flask and shaking for 40 minutes. In a blank experiment no lipase is added. After washing, the swatch is rinsed with STW and subsequently dried at 55°C for one hour after which a second washing cycle is carried out. The dried swatch is extracted by rotation in a glass tube containing 5 ml of solvent having the same composition as the eluent used for the chromatographic separation of substrate and products.

In the extraction solution the residual amount of triglyceride together with the amount of free fatty acid and 1,2 and 1,3-diacylglycerides formed are determined by HPLC.

### Equipment and conditions

| | |
|---|---|
| Pump | LKB (model 2150) |
| Detection | Refractive index monitor (Jobin Yvon) |
| Injection system | Perkin-Elmer ISS-101; 10 µl |
| Integrator | Spectra Physics, Chromjet |
| Column | CP Microspher-Si (Chrompack), 100x4.6 mm |
| Eluent | n-hexane/isopropylalcohol/formic acid: 975:25:2.5 (v/v), 1 ml/min. |
| Temperature | ambient |

Under these conditions the retention times of triolein, oleic acid, 1,3 and 1,2-diacylglyceride are 1.2, 1.6, 2.4 and 3.4 minutes, respectively. The peak areas or peak heights are measured. They are a measure of the recovery of the triglyceride, free fatty acid and diacylglyceride after extraction from the swatch. The recovery of triglyceride after extraction from an unwashed swatch is taken as 100%. Under the conditions described above the ratio of the refractive index responses between olive oil, oleic acid, 1,2 and 1,3-diacylglyceride was found to be 1.00, 0.98, 2.10 and 1.30, respectively, on the basis of peak height.

### Assay for the determination of lipase activity

Activities of the lipase and mutants of the invention, expressed as ILU's, were determined on the basis of hydrolysis of olive oil. The hydrolysis is measured at 30°C, in a pH-stat containing 10% olive oil in a 0.4 mM Tris buffer pH 9 in the presence of 20 mM sodium chloride and 10 mM calcium chloride. One ILU is defined as the amount of enzyme needed for the release of one µmole fatty acid per minute under the conditions of the test.

The following examples are offered by way of illustration and not by way of limitation.

### Example 1

### Development of a mutagenesis system for the lipase gene

Current strategies for site-directed mutagenesis can be divided into two categories:
1) mutagenic primer-directed ill-in of a gapped duplex DNA will yield a heteroduplex DNA, containing one mutated strand
2) the appropriately mutated sequence may be introduced by replacing a cassette within the target gene. A PCR fragment, containing the desired mutation, is then exchanged in the expression cassette.

### Site directed mutagenesis using PCR

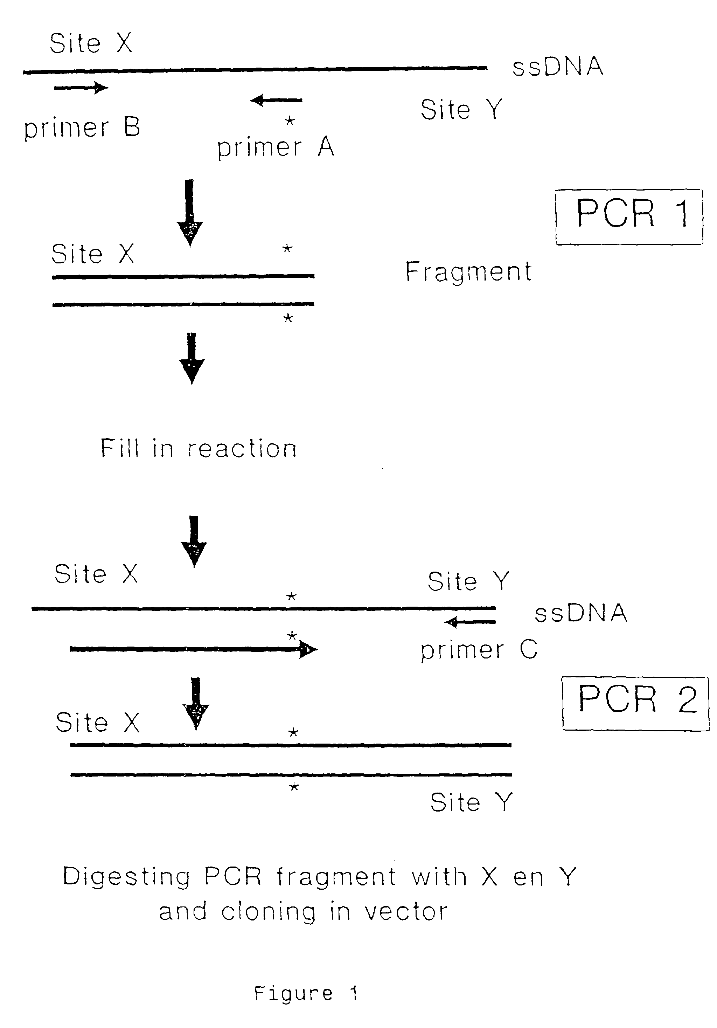

This method was derived from Xiong, who presented a general description of the method at the third Pseudomonas meeting 1991. The method is schematically drawn in Figure 1.

For the isolation of single stranded DNA we used plasmid pTMPv18A, which was described in EP 0334462. For the amplification reaction Vent-polymerase (Biolabs) was used. This polymerase contains 3' proofreading activity and therefore misincorporations due to PCR reaction are decreased.

PCR reaction was carried out in the following buffer containing: 10 mM KCl, 10 mM (NH₄)₂-SO₄, 20 mM Tris-HCl pH 8.8, 2 mM MgSO₄ and 0.1% Triton X-100, 0.2 mM dATP, 0.2 mM dCTP, 0.2 mM dGTP, 0.2 mM dTTP and 1 unit Vent polymerase.

In step 1, 0.5 µM of primer A, containing the mutation, 0.05 µM primer B and 1-10 ng of single stranded pTMPv18A was added to 50 µl reaction mixture and 25 cycles consisting of 2 minutes at 98°C, 2 minutes at 55°C and 2 minutes at 72°C were carried out.

Then Klenow DNA polymerase and ligase was added and the single stranded DNA was filled in, using the fragment containing the mutation as a primer. This will yield a heteroduplex DNA, containing one mutated strand. Furthermore the mixture was treated with exonuclease in order to remove single stranded DNA and primers A and B. DNA was then precipitated and resolved in PCR reaction buffer. The newly synthetized DNA strand, containing the mutation, was used as a template in step 2.

In step two 0.05 µM of primer C was added to this mixture. A second PCR was performed as described.

The PCR fragment is then digested with suitable restriction enzymes and subcloned in the integration vector.

### Example 2

### Introduction of mutation near active site: changing position M21

Mutations were introduced as described above. XhoI and BclI were selected as suitable restriction enzymes. The oligonucleotides which were used, are shown in Table 2:

**Table 2**

| | | | |
|---|---|---|---|
| Oligo 5' → 3' | Primer code | Mutation | Position |
| CTGCGGGCTGGTGCTGGAGCTGCC | Primer C | - | 829-852 R |
| GGATGCAAGGATGGATCAGTGCCC | Primer B | - | 266-280 |
| CGAAGCCGAGNNNGCCGTGGGTC | Primer A | M21X | 426-450 R |

### Example 3

### Chromosomal inactivation of the lipase gene of Pseudomonas pseudoalcaligenes M1 (CBS 473.85)

A suicidal integration plasmid, which is unable to replicate in Pseudomonas pseudoalcaligenes, but able to replicate in other microorganisms, was used to inactivate the lipase gene in the chromosome of Pseudomonas pseudoalcaligenes.

The lipase containing gene fragment was subcloned from plasmid pTMPv18 on plasmid pBR322 (Bolivar et al. Gene 2 (1977) 95-113), which is able to replicate in E. coli, but unable to replicate in Pseudomonas pseudoalcaligenes. Then an internal fragment was deleted from the plasmid. The resulting plasmid was called pBRflank.

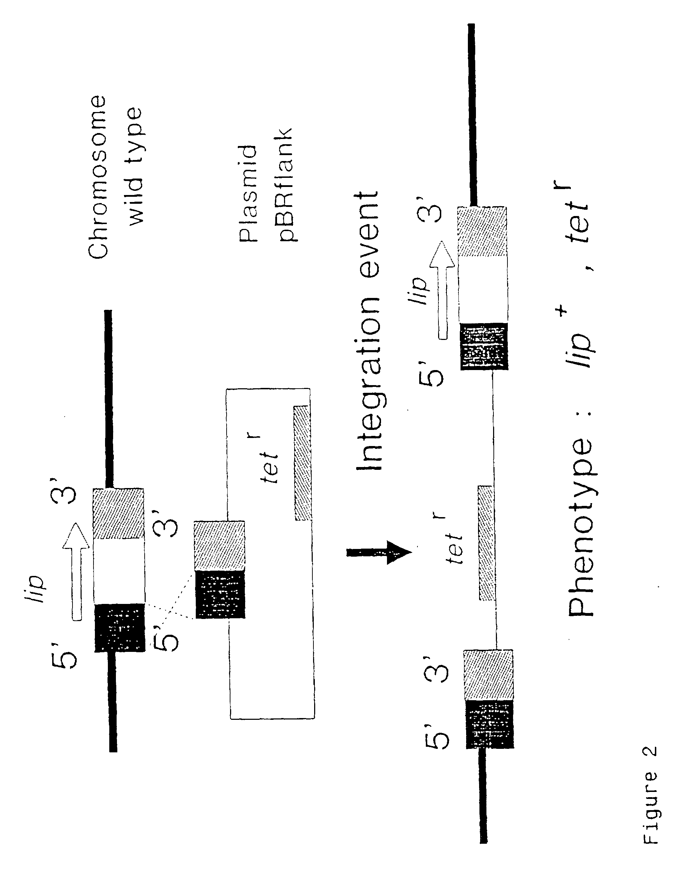

Pseudomonas pseudoalcaligenes M1 (CBS 473.85) was transformed with pBRflank. Since this plasmid is unable to replicate in Pseudomonas, tetracycline resistant colonies can only be obtained by integration. Several tetracycline resistant (5 mg/l) colonies were selected. In these strains the plasmid pBRflank is integrated into the bacterial chromosome by a single recombination event at the 5' or 3' flanking region (Figure 2). Due to the fact that these strains still contain a functional lipase gene, they harbour a lipase positive and tetracycline^{R} phenotype. Several strains were selected for further experiments. In order to delete the lipase gene and the plasmid from the chromosome, a second recombination (excision) event has to occur. This can be achieved by growing the strains for several days in BHI (Brain Heart Infusion) medium, in the absence of antibiotics.

Then the cells were plated on agar medium, containing tributyrin. The colonies containing a lipase negative phenotype were also tested for their unability to grown on selective agar plates. The resulting lipase negative strain was called Ps600.

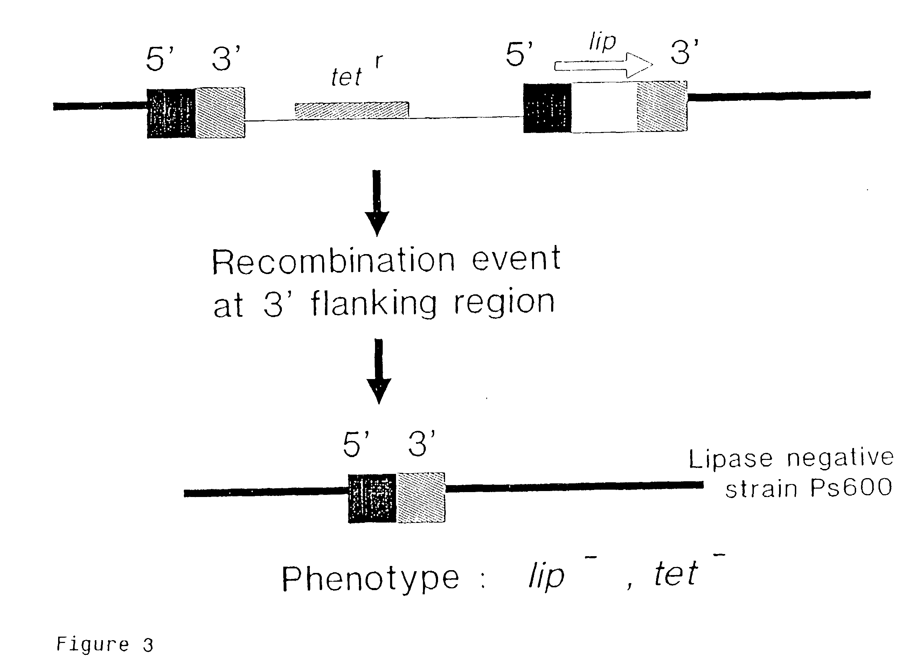

A schematic view of this integration event, followed by a second recombination is shown in Figures 2 and 3.

### Introduction of a mutant lipase gene in the lipase negative strain Pseudomonas pseudoalcaligenes Ps600

In order to produce high amounts of the mutant enzymes, the mutated genes were both integrated into the chromosome and introduced into the same strain on a plasmid. Integration of the mutant lipase genes in the chromosome was obtained in a similar way as described in the previous example.

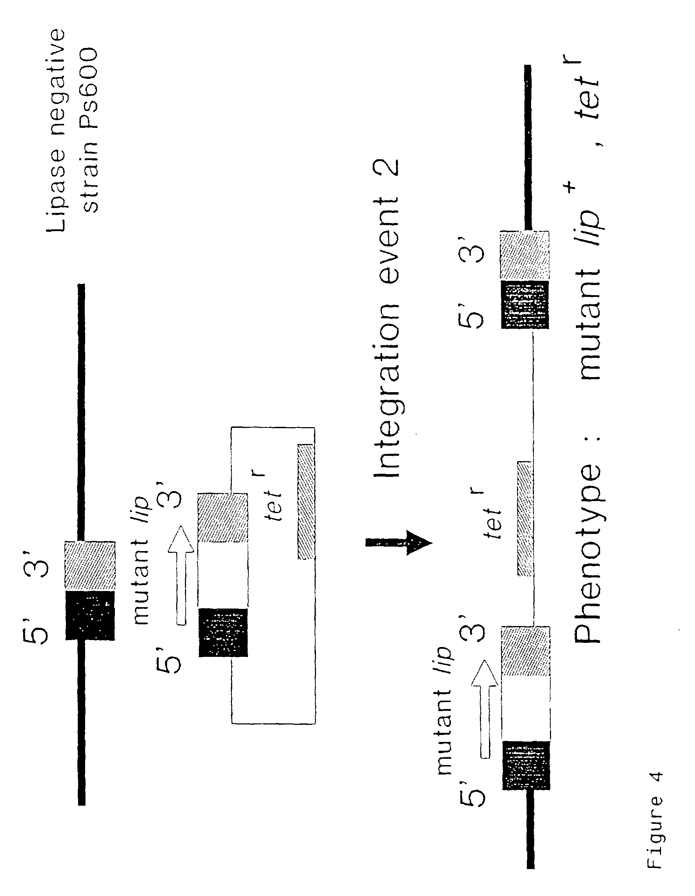

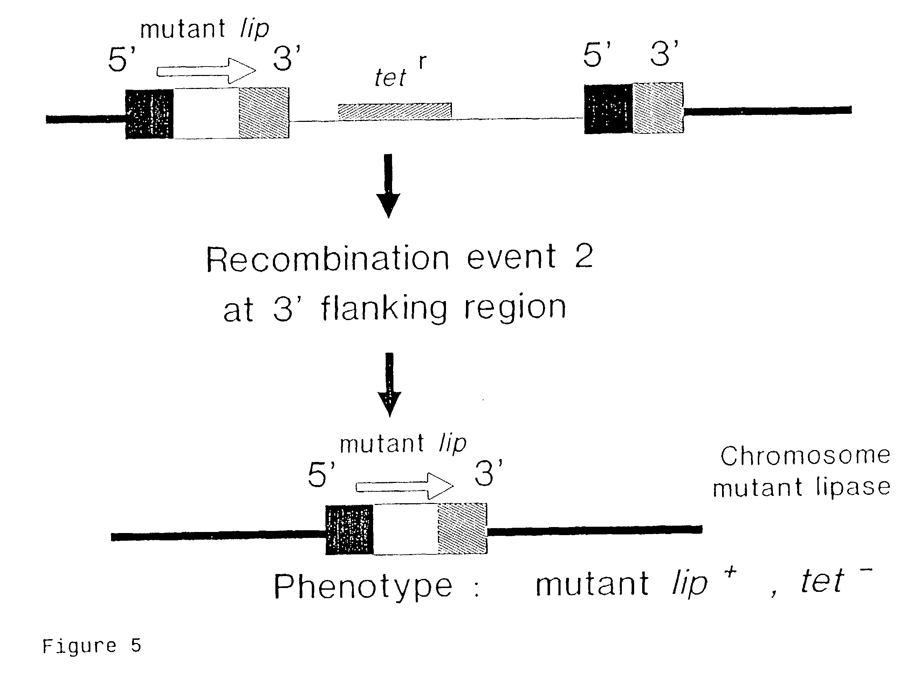

A schematic draft of this event is shown in Figures 4 and 5.

### Production of the mutant lipases

The strains were grown as described in EP 0334462. THe lipase protein was then purified from the culture broth. This procedure has also been described in EP 0334462.

### Example 4

### Determination of the specific activity of the M21L mutant lipase

Using the activity assay previously mentioned and the quantitative amino acid analysis for the quantification of the protein content, the specific activity of both wild-type and mutant was determined. The specific activity of the mutant is a factor 1.4 less than wild-type enzyme: 8000 versus 6390 ILU/mg protein.

### Example 5

### Lipase activity of wild-type lipase M1 (CBS 473.85) and some M21 mutants under application conditions (SLM test)

The SLM test was carried out as described herein before. Both wild-type and mutants were tested in a single and two cycle washing test under the following conditions:
- standard tap water (STW)
- detergent is Ariel Ultra™ (2 g/l)
- lipase dosage as indicated
Ariel Ultra™ is a product of Procter & Gamble and is commercially available. This detergent contains neither a protease nor a lipase.

These conditions represent essentially the European wash conditions.

**Table 3**

| Percentage residual triglyceride after one and two wash cycles in the presence and absence of lipase M1 and some M21 mutants | | | |
|---|---|---|---|
| Lipase | conc. (µg/ml) | residual triglyceride (%) after | |
| | | one cycle | two cycles |
| M1 | 0 | 82 | 58 |
| M1 | 0.23 | 71 | 45 |
| M1 | 0.45 | 68 | 37 |
| M1 | 0.68 | 60 | 29 |
| M1 | 0.91 | 60 | 20 |
| M21L | 0 | 84 | nd |
| M21L | 0.16 | 58 | nd |
| M21L | 0.31 | 43 | nd |
| M21L | 0.47 | 39 | nd |
| M21L | 0.63 | 34 | 2 |
| M21L | 1.25 | 32 | 2 |
| M21A | 0.25 | 60 | 28 |
| M21A | 0.50 | 56 | 16 |
| M21A | 0.75 | 42 | 11 |
| M21A | 1.00 | 35 | 7 |
| M21A | 1.50 | 24 | 2 |
| M21A | 2.00 | 23 | 0 |
| nd = not determined | | | |

From this Table it appears that the lipases used show their lipolytic properties on textile. These results clearly demonstrate that the M21L and M21A mutants are more active than the wild-type enzyme under these application conditions when added on the same weight base.

### Example 6

### Performance of wild-type lipase (M1) and mutant M21L in a washing process according to the SLM test

The compatibility of these enzymes with a powder detergent and a bleach system was checked under the following conditions:
- standard hardness water (0.75 mM of calcium and 0.25 mM of magnesium)
- detergent is Tide™ (1 g/l)
- lipase dosage as indicated
- two cycles wash test.

Tide™ is a product of Procter & Gamble and is commercially available. This detergent contains a protease but no lipase.

These conditions represent essentially the U.S. wash conditions.

From Table 4 it clearly appears that the lipases used in this example show their lipolytic properties on textile, and, in particular, their wash performance in powder detergent. The wash performance of the mutant enzyme is superior to that of the wild-type lipase in a detergent with and without bleach.

**Table 4**

| Wash performance of lipase M1 and mutant M21L | | | | | | |
|---|---|---|---|---|---|---|
| lipase | conc. (µg/ml) | bleach* | recovery (%) | | | |
| | | | TG** | DG | FFA | Total |
| M1 | 0 | - | 100 | 0 | 0 | 100 |
| M1 | 0.23 | - | 89 | 3 | 2 | 94 |
| M1 | 0.45 | - | 63 | 8 | 9 | 80 |
| M1 | 0.68 | - | 62 | 9 | 12 | 83 |
| M1 | 0.91 | - | 50 | 10 | 16 | 76 |
| M1 | 0 | + | 90 | 0 | 0 | 90 |
| M1 | 0.23 | + | 95 | 0 | 0 | 95 |
| M1 | 0.45 | + | 98 | 0 | 0 | 98 |
| M1 | 0.68 | + | 94 | 0 | 0 | 94 |
| M1 | 0.91 | + | 97 | 1 | 1 | 99 |
| M21L | 0 | - | 102 | 0 | 0 | 102 |
| M21L | 0.08 | - | 92 | 4 | 1 | 97 |
| M21L | 0.16 | - | 81 | 5 | 5 | 91 |
| M21L | 0.31 | - | 43 | 8 | 17 | 68 |
| M21L | 0.47 | - | 35 | 8 | 21 | 64 |
| M21L | 0.63 | - | 33 | 8 | 20 | 61 |
| M21L | 0.94 | - | 29 | 7 | 20 | 56 |
| M21L | 1.25 | - | 24 | 6 | 23 | 53 |
| M21L | 0 | + | 98 | 0 | 0 | 98 |
| M21L | 0.08 | + | 78 | 5 | 5 | 88 |
| M21L | 0.16 | + | 50 | 8 | 15 | 73 |
| M21L | 0.31 | + | 39 | 9 | 20 | 68 |
| M21L | 0.47 | + | 33 | 8 | 22 | 63 |
| M21L | 0.63 | + | 29 | 7 | 21 | 57 |
| M21L | 0.94 | + | 23 | 8 | 23 | 54 |
| M21L | 1.25 | + | 14 | 6 | 23 | 43 |

| | | | | | | |
|---|---|---|---|---|---|---|
| * bleach system (concentration used is 0.3 g/l) is perborate/NOBS (6.6:1); NOBS stands for Nonanoyloxy Benzene Sulphonate | | | | | | |
| ** TG = triglyceride, DG = diglyceride, FFA = free fatty acid | | | | | | |

### Example 7

### Performance of wild-type lipase M1 (CBS 473.85) and the M21A mutant in a washing process according to the SLM test

The wash performance and the compatibility of these enzymes with a powder detergent and a bleach system was checked under the following conditions:
- standard hardness water (0.75 mM of calcium and 0.25 mM of magnesium)
- detergent is Tide™ (1 g/l)
- lipase dosage as indicated
- two cycles wash test.

Tide™ is a product of Procter & Gamble and is commercially available. This detergent contains a protease but no lipase. The swatch used in this wash test comes from a different batch with a different behaviour in the wash.

These conditions represent essentially the U.S. wash conditions.

From Table 5 it clearly appears that the lipases used in this example show their lipolytic properties on textile, and their wash performance in a detergent with and without bleach.

The wash performance of the M21A mutant is improved in comparison to that of the wild-type.

**Table 5**

| Wash performance of lipase M1 and mutant M21A | | | | | | |
|---|---|---|---|---|---|---|
| lipase | conc. (µg/ml) | bleach* | recovery (%)** | | | |
| | | | TG | DG | FFA | Total |
| M1 | 0 | - | 35 | 0 | 0 | 35 |
| M1 | 0.23 | - | 24 | 0 | 5 | 29 |
| M1 | 0.45 | - | 16 | 0 | 5 | 21 |
| M1 | 0.68 | - | 16 | 0 | 6 | 22 |
| M1 | 0 | + | 34 | 0 | 0 | 34 |
| M1 | 0.23 | + | 20 | 0 | 2 | 22 |
| M1 | 0.68 | + | 15 | 0 | 1 | 16 |
| M21A | 0 | - | 35 | 0 | 0 | 35 |
| M21A | 0.13 | - | 23 | 0 | 6 | 29 |
| M21A | 0.25 | - | 15 | 0 | 6 | 21 |
| M21A | 0.50 | - | 16 | 0 | 8 | 24 |
| M21A | 0.75 | - | 15 | 1 | 11 | 27 |
| M21A | 0 | + | 34 | 0 | 0 | 34 |
| M21A | 0.13 | + | 16 | 0 | 4 | 20 |
| M21A | 0.25 | + | 14 | 0 | 5 | 19 |
| M21A | 0.50 | + | 11 | 0 | 6 | 17 |
| M21A | 0.75 | + | 10 | 0 | 8 | 18 |

| | | | | | | |
|---|---|---|---|---|---|---|
| * bleach system (concentration used is 0.3 g/l) is perborate/OBS (6.6:1); NOBS stands for Nonanoyloxy Benzene Sulphonate | | | | | | |
| ** TG = triglyceride, DG = diglyceride, FFA = free fatty acid | | | | | | |

### Example 8

### Lipase activity of wild-type M1 lipase and M21L mutant under application conditions (SLM test) at different temperatures

The SLM test was carried out as described hereinbefore. Both wild-type and mutant were tested in a single washing test under the following conditions:
- standard tap water (STW)
- detergent is Ariel Ultra™ (2 g/l)
- lipase dosage as indicated

Ariel Ultra is a product of Procter & Gamble and is commercially available. It contains no enzymes.

**Table 6**

| Percentage residual triglyceride after one wash cycle at different temperatures in the absence and presence of M21L mutant and wild-type lipase M1. | | | | |
|---|---|---|---|---|
| Lipase | conc. (µg/ml) | residual TG* (%) | after a single wash at T (°C) | |
| | | 40 | 50 | 60 |
| - | 0 | 75 | 85 | 71 |
| M1 | 0.21 | 60 | 66 | 71 |
| M1 | 0.43 | 55 | 64 | 68 |
| M1 | 0.64 | 55 | 56 | 78 |
| M1 | 0.85 | 46 | 53 | 72 |
| M21L | 0.20 | 47 | 48 | 65 |
| M21L | 0.41 | 42 | 35 | 59 |
| M21L | 0.61 | 36 | 25 | 56 |
| M21L | 0.81 | 36 | 25 | 53 |

| | | | | |
|---|---|---|---|---|
| * TG = triglyceride | | | | |

This Example clearly demonstrates that at all temperatures tested the lypolytic capacity of the mutant is superior over that of the wild-type under the application conditions of the test. Where the temperature optimum (for a maximum of triglyceride hydrolysis) of lipase M1 in this test under these application conditions is found at 40°C, that of the mutant is shifted towards a higher value: 50°C.

### EXAMPLE 9

### Shelf stability of formulated lipase M1 and some M21 mutants

Lipase M1 and the M21L and M21A mutants were formulated in a PEG-prill. These granules were then stored in closed glass tubes at temperatures indicated in the Table 7 below. After 8 weeks of storage the residual activity of the lipases was measured in the pH-stat.

As can be seen from the table, all lipases contained after 8 weeks of storage at a temperature up to 37°C a residual activity of over 90 % of its original.

**Table 7**

| Storage stability of lipase M1 and various M21 mutants | | | |
|---|---|---|---|
| lipase | residual activity (%) after storage at temp.(°C) | | |
| | 4 | 25 | 37 |
| lipase M1 | 98 | 95 | 95 |
| M21L mutant | 97 | 102 | 102 |
| M21A mutant | 93 | 95 | 95 |

The new lipase variants according to the present invention are particularly useful in detergent applications, such as laundry washing and dish washing, but they may also be used for other applications known in the art. The lipases are preferably used as a detergent additive, either in particulate (e.g. prills) or in liquid form, usually with a stabilizer. The replacement of Met +21 has a positive effect on detergent stability, the extent being dependent on the specific amino acid used. Also the stability of Met +21 mutants in solution, both in the presence and absence of a bleach system (e.g. perborate/ TAED) is surprisingly good. The lipases may be applied together with other enzymes, such as amylases, proteases, cellulases and various cell-wall degrading enzymes.

All publications (including patents and patent applications) mentioned in this specification are indicative to the level of skill of those skilled in the art to which this invention pertains.

## Claims

1. A modified lipase from a Group I Pseudomonas comprising an amino acid sequence in which the methionine at a position corresponding to methionine 21 of wild-type Pseudomonas pseudoalcaligenes lipase is substituted by leucine or alanine.

2. A modified lipase according to claim 1, wherein the wild-type lipase has at least 70% homology with Pseudomonas pseudoalcaligenes lipase.

3. A modified lipase according to claim 1 wherein the Group I Pseudomonas is either Pseudomonas pseudoalcaligenes or Pseudomonas aeruginosa.

4. A modified lipase according to any one of claims 1 to 4, which shows an improved wash performance compared to the corresponding wild-type lipase.

5. A modified lipase according to claim 4, wherein the improvement in wash performance is determined by the SLM test.

6. A detergent composition comprising a modified lipase according to any one of claims 1 to 5.

7. Use of a modified lipase according to any one of claims 1 to 6, as a detergent ingredient.

8. A recombinant DNA sequence encoding a modified lipase according to any one of claims 1 to 5.

9. A recombinant DNA vector comprising the recombinant DNA sequence of claim 8 and which is useful in the expression of the modified lipase.

10. A transformed host microorganism comprising a recombinant DNA sequence according to claim 8 or a recombinant DNA vector according to claim 9.

11. A transformed host microorganism according to claim 10 which is a Pseudomonas.

12. A process which comprises effecting a mutation in DNA encoding a Pseudomonas Group I lipase at the methionine at a position corresponding to methionine 21 in Pseudomonas pseudoalcaligenes lipase, which mutation is a substitution by leucine or alanine, in order to improve the wash performance of said lipase.

## Patentansprüche

1. Modifizierte Lipase von Gruppe I Pseudomonas, umfassend eine Aminosäuresequenz, worin das Methionin an einer Stellung korrespondierend zu Methionin 21 von Wildtyp Pseudomonas pseudoalcaligenes Lipase durch Leucin oder Alanin substituiert ist.

2. Modifizierte Lipase gemäß Anspruch 1, wobei die Wildtyplipase mindestens 70 % Homologie mit Pseudomonas pseudoalcaligenes Lipase aufweist.

3. Modifizierte Lipase gemäß Anspruch 1, wobei der Gruppe I Pseudomonas entweder Pseudomonas pseudoalcaligenes oder Pseudomonas aeruginosa ist.

4. Modifizierte Lipase gemäß einem der Ansprüche 1 bis 4, die eine verbesserte Waschleistung im Vergleich zu der korrespondierenden Wildtyplipase zeigt.

5. Modifizierte Lipase gemäß Anspruch 4, wobei die Verbesserung der Waschleistung durch den SLM-Test bestimmt wird.

6. Detergenszusammensetzung, umfassend eine modifizierte Lipase gemäß einem der Ansprüche 1 bis 5.

7. Verwendung einer modifizierten Lipase gemäß einem der Ansprüche 1 bis 6 als Detergensbestandteil.

8. Rekombinante DNA-Sequenz, kodierend eine modifizierte Lipase gemäß einem der Ansprüche 1 bis 5.

9. Rekombinanter DNA-Vektor, umfassend die rekombinante DNA-Sequenz gemäß Anspruch 8, der für die Expression der modifizierten Lipase geeignet ist.

10. Transformierter Wirts-Mikroorganismus, umfassend eine rekombinante DNA-Sequenz gemäß Anspruch 8 oder einen rekombinanten DNA-Vektor gemäß Anspruch 9.

11. Transformierter Wirts-Mikroorganismus gemäß Anspruch 10, wobei es sich um Pseudomonas handelt.

12. Verfahren, das die Bewirkung einer Mutation in einer DNA, kodierend eine Pseudomonas-Gruppe I-Lipase an dem Methionin an einer Stellung, korrespondierend zu Methionin 21 bei Pseudomonas pseudoalcaligenes-Lipase umfasst, wobei die Mutation eine Substitution durch Leucin oder Alanin ist, um die Waschleistung der Lipase zu verbessern.

## Revendications

1. Lipase modifiée d'un *Pseudomonas* du Groupe I comprenant une séquence d'acides aminés dans laquelle la méthionine occupant une position correspondant à la méthionine 21 de la lipase de *Pseudomonas pseudoalcaligenes* de type sauvage est remplacée par la leucine ou l'alanine.

2. Lipase modifiée selon la revendication 1, dans laquelle la lipase de type sauvage présente une homologie d'au moins 70% avec la lipase de *Pseudomonas pseudoalcaligenes.*

3. Lipase modifiée selon la revendication 1, dans laquelle le *Pseudomonas* du Groupe I est *Pseudomonas pseudoalcaligenes* ou *Pseudomonas aeruginosa.*

4. Lipase modifiée selon l'une quelconque des revendications 1 à 4, laquelle manifeste une performance de lavage améliorée par rapport à la lipase de type sauvage.

5. Lipase modifiée selon la revendication 4, dans laquelle l'amélioration de la performance de lavage est déterminée à l'aide du test "SLM".

6. Composition détergente comprenant une lipase modifiée selon l'une quelconque des revendications 1 à 5.

7. Utilisation d'une lipase modifiée selon l'une quelconque des revendications 1 à 6, en qu'un ingrédient détersif.

8. Séquence d'ADN recombinante codant pour une lipase modifiée selon l'une quelconque des revendications 1 à 5.

9. Vecteur d'ADN recombinant comprenant la séquence d'ADN recombinante selon la revendication 8 et qui est utile dans l'expression de la lipase modifiée.

10. Micro-organisme hôte transformé comprenant une séquence d'ADN recombinante selon la revendication 8 ou un vecteur d'ADN recombinant selon la revendication 9.

11. Micro-organisme hôte transformé selon la revendication 10 qui est un *Pseudomonas.*

12. Procédé qui comprend la réalisation d'une mutation dans l'ADN codant pour une lipase d'un *Pseudomonas* du Groupe I portant sur le résidu méthionine occupant une position correspondant à la méthionine 21 de la lipase de *Pseudomonas pseudoalcaligenes,* laquelle mutation est une substitution par la leucine ou l'alanine, destinée à améliorer la performance de lavage de ladite lipase.


### SEQUENCE LISTING

(1) GENERAL INFORMATION:
   (i) APPLICANT:
      (A) NAME: GIST-BROCADES N.V.
      (B) STREET: P.O. Box 1
      (C) CITY: Delft
      (E) COUNTRY: Netherlands
      (F) POSTAL CODE (ZIP): NL-2600 MA
      (G) TELEPHONE: 31.15.791111
      (H) TELEFAX: 31.15.793957
   (ii) TITLE OF INVENTION: NEW LIPASE VARIANTS FOR USE IN DETERGENT APPLICATIONS
   (iii) NUMBER OF SEQUENCES: 3
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: EP 93201212.3
      (B) FILING DATE: 27-APR-1993
(2) INFORMATION FOR SEQ ID NO: 1:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: primer A
   (xi) SEQUENCE DESCRIPTION: SEQ ID NO: 1:
   CGAAGCCGAG NNNGCCGTGG GTC 23
(2) INFORMATION FOR SEQ ID NO: 2:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: primer B
   (xi) SEQUENCE DESCRIPTION: SEQ ID NO: 2:
   GGATGCAAGG ATGGATCAGT GCCC 24
(2) INFORMATION FOR SEQ ID NO: 3:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (synthetic)
   (iii) HYPOTHETICAL: NO
   (vi) ORIGINAL SOURCE:
      (C) INDIVIDUAL ISOLATE: primer C
   (xi) SEQUENCE DESCRIPTION: SEQ ID NO: 3:
   CTGCGGGCTG GTGCTGGAGC TGCC                                24